# EUROPEAN PATENT SPECIFICATION

(11) **EP 2 403 764 B1**
(45) Date of publication and mention of the grant of the patent: **19.03.2014**
(21) Application number: 10707648.1
(22) Date of filing: 01.03.2010
(51) Int. Cl.: B65F 1/02, B65F 1/10, B65D 5/36, B65D 83/08

(54) **STORAGE BOX**
LAGERUNGSKASTEN UND ZUGEHÖRIGER ZUSCHNITT
BOITE DE STOCKAGE ET FLAN ASSOCIE

(30) Priority: 04.03.2009 GB 0903703
(43) Date of publication of application: 11.01.2012
(73) Proprietor: Seiquelle Innovation Limited, Hungerford, Berkshire RG17 0NB (GB)
(72) Inventor: HOFGARTNER, Susan, Hungerford Berkshire RG17 0NB (GB)
(74) Representative: Humphrey-Evans, Edward John
(86) International application number: PCT/GB2010/050354
(87) International publication number: WO 2010/100478

(56) References cited:
- DE-U1- 8 908 894
- GB-A- 2 135 277
- US-A1- 2008 314 921

## Description

The present invention relates to storage boxes and, more particularly, to storage boxes constructed of flexible material such as card which are foldable into a configuration whereby they may be more readily and easily stored, e.g. they are collapsible.

Such foldable storage boxes generally comprise front, rear and side panels together with at least one base panel. The side panels are generally scored or otherwise foldable along at least a part of their length, enabling them to be pushed inwards and thus permit the front and rear panels to be brought closer to one another for at least partial collapse and storage of the box. The rear panel may also be foldable to permit more complete collapse of the box to enable it to be stored flat.

In use, the side panels are unfolded out into their original position thus separating the front and rear panels to form a storage box.

A disadvantage attendant on such boxes is that resiliently deformable materials such as card may, once folded, have a tendency to revert to the folded position. This is particularly so where a box has been stored in a folded position for some length of time. The result is that the side panels and rear panel tend to fold back to their position as stored, so collapsing (at least partially) the box.

It is from a consideration of such storage boxes and their attendant disadvantages that the present invention has been developed.

In accordance with a first aspect of the present invention, there is provided a collapsible box according to claim 1.

In their first position, the wing portion fold lines are complementary with the fold lines of the side panels. Advantageously, in the second position the wing portions will act to resist compression or collapse of the box, preferably through rigidity of the hinge and/or through the longitudinal fold lines of the wing portions. Preferably the box further comprises a top panel comprising an aperture, the wing portions being foldable over this top panel and preferably being shaped to further define the edges of the aperture. The top panel is preferably also foldable to permit collapse of the box.

Preferably the top panel further comprises a flap at least partially occluding the aperture.

Preferably the box further comprises a lid panel moveable between a first closed position overlying and occluding the aperture and a second open position exposing the flap, the lid panel preferably being interoperably connected with the flap such that in the first closed position it will act to limit occlusion of the aperture by the flap.

The lid panel preferably comprises a handle portion.

In such a configuration, the contents of the box will preferably be hidden from view whether the lid is open or closed, thus making the box suitable for storage/disposal of e.g. sanitary towels. Rather than empty the box for re-use it may simply be disposed of.

In such a configuration the material forming the box should be waterproof or otherwise treated to resist water.

Preferably the wing portions are foldable underneath the lid panel or otherwise configured so as not to interfere with movement of the lid panel between its first and second positions, when the wing portions are in the second position. Ideally the wing portions will define the edges of the aperture so as to prevent the flap portion from moving above the top of the box.

In accordance with an associated aspect of the invention, there is provided a disposable sanitary waste box, having a configuration as described above.

In another aspect of the present invention there is provided a blank for forming a collapsible box, according to claim 9.

The second panel may be foldable along its width to permit an assembled box to be collapsed and stored flat.

Preferably the further panels are shaped such that in the second position they can define an aperture permitting access to the assembled box.

Preferably the blank further comprises a further panel hingedly connected with the first or second panel and defining an aperture, the panel being foldable around the first or second panel to form a top panel for the assembled box.

Preferably the further panel comprises a flap at least partially occluding the aperture.

Preferably the blank further comprises a further panel hingedly connected with the second or first panel, the panel being foldable around the second or first panel to form a lid panel for the assembled box.

The connection of the further panel may depend on which panel the top-forming panel is connected to.

Preferably the further panel comprises a first part shaped in use to occlude the aperture of the top-forming panel and a second part foldable to form a handle portion.

The further panel may comprise further portions hingedly connected to its first part to fold around and so reinforce the first part.

The blank may comprise a plurality of panels hingedly connected to any or all of the first, second, third and fourth panels and foldable around them to form a base for the assembled box.

The box and/or blank may also be treated to resist cleaning chemicals, and/or may be treated to have antimicrobial properties. Additionally or alternatively the material may be treated with a fragrance material, or the box may be adapted to receive a fragrance emitter. Alternatively, the box and/or blank may be made from a material which is resistant to one or more of water and cleaning chemicals and solutions. The material may have or may have been imbued with antimicrobial properties and/or may have or be impregnated with a fragrance.

The box and/or blank may be made from laminated sheets, e.g. sheets of card, cardboard or the like.

Accordingly, blanks may be provided as flat bodies, whose major surfaces may be water and/or chemical resistant, e.g. laminated, but the edges may comprise exposed, untreated material such as card or cardboard.

The areas of exposed, untreated material may be treated to provide the blank and/or box with enhanced water and/or chemical resistance. For instance, a waterproof silicone compound such as may be used to waterproof leather may be applied, e.g. by spraying using a butane propellant.

Conveniently, a stack comprising a plurality of blanks may be treated in this way simultaneously.

It has been found that following spraying, the sprayed compound typically penetrates the exposed card or cardboard, but does not cause discolouration.

The box and/or blank may be provided in a range of colours. Accordingly, the box may be more visible and/or more aesthetically pleasing in or sympathetic to its intended site-of-use. For instance, a given colour may signify, in use, that a box is to be used for receiving a specific type of waste. In some preferred embodiments, at least a portion of the box and/or blank may be pink in colour.

Conveniently the blank for forming a collapsible box further comprises a fourth panel hingedly connected to the first panel or the second panel.

In order that the invention may be more readily understood, better appreciated and more easily put into effect, reference will now be made by way of non-limitative example to the accompanying drawings, wherein:
Figure 1 shows a plan view of the blank template ready for assembly;
Figure 2 shows a rear view of the assembled box;
Figure 3 is a cut-away plan view from the top of the box once assembled showing a detail of the wings;
Figure 4 shows a view of the base of the assembled box;
Figure 4a shows a detail of Figure 4;
Figure 5 shows a side view of the assembled box;
Figure 6 is a detail view of the top of the assembled box showing construction of the lid;
Figure 7 shows an isometric view of the assembled box;
Figure 8 shows a view of the right side of the box part-way through construction;
Figure 9 shows a view of the left side of the box part-way through construction; and
Figure 10 shows the box in a partially collapsed state.

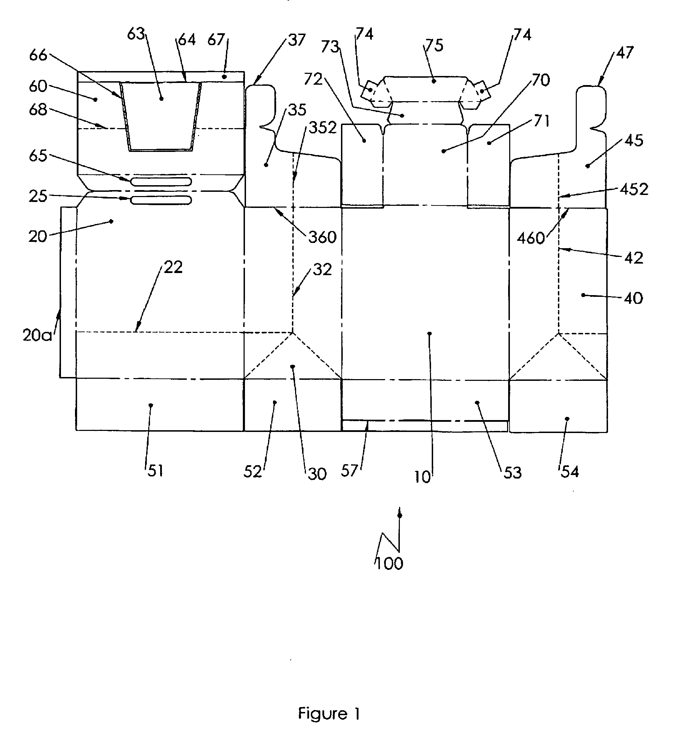

Referring now to Figure 1, the present invention comprises a blank 100 foldable into a storage box and having a first panel 10 and a second panel 20 together with a third panel 30 and a fourth panel 40. Further panels 51 to 54 will form the base of the box once assembled. The panels 10, 20, 30, 40 and 51 to 54 are hingedly connected and hence foldable around the intermittent dotted lines shown. The second panel 20 may be provided with an aperture 25 which will serve as a handle for the assembled box.

The blank 100 is also provided with a ninth panel 60 which is likewise foldable around the intermittent dotted lines and is thus hingedly connected with the rear panel 20.

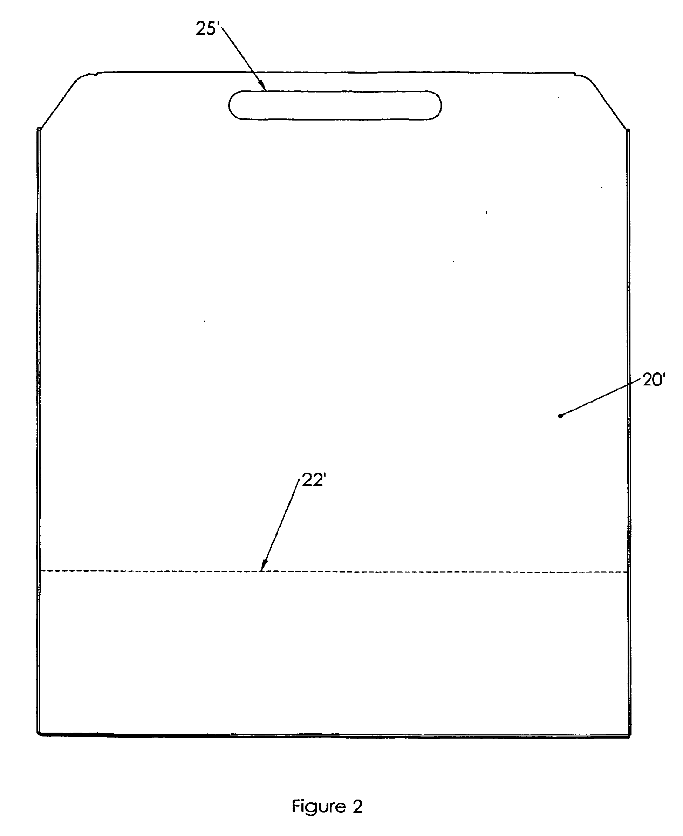

Where the second panel 20 (foldable to form the rear panel 20' of a box, shown in Figure 2) is provided with an aperture 25, the ninth panel 60 may be likewise provided with a complementary aperture 65 which will line up with the aperture 25 of the second panel 20 to form a handle 25' (shown in Figure 2) once the box is assembled. The ninth panel 60 may alternatively be hingedly connected to the first panel 10.

Referring back to Figure 1, the ninth panel 60 is foldable over the second panel to form a top panel of a box. The ninth panel 60 contains an aperture 66 through which items may be placed in the assembled box. Preferably, however and as described in more detail below, the ninth panel further comprises a flap 63 hingedly connected, along a fold line 64, with the ninth panel 60 and which at least partially occludes the aperture 66. The flap 63 may be reinforced, e.g. by an additional layer of card, which may be included with the box together with an appropriate adhesive.

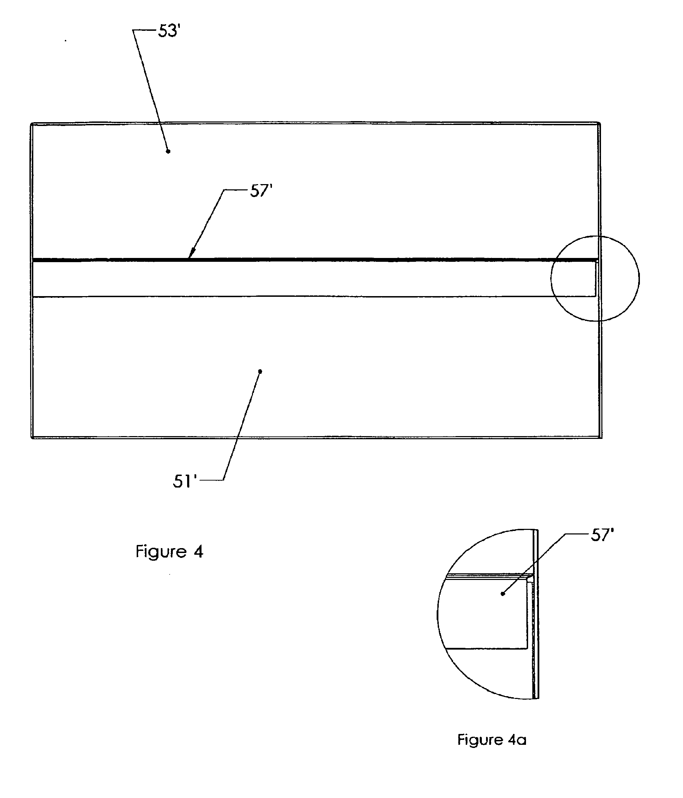

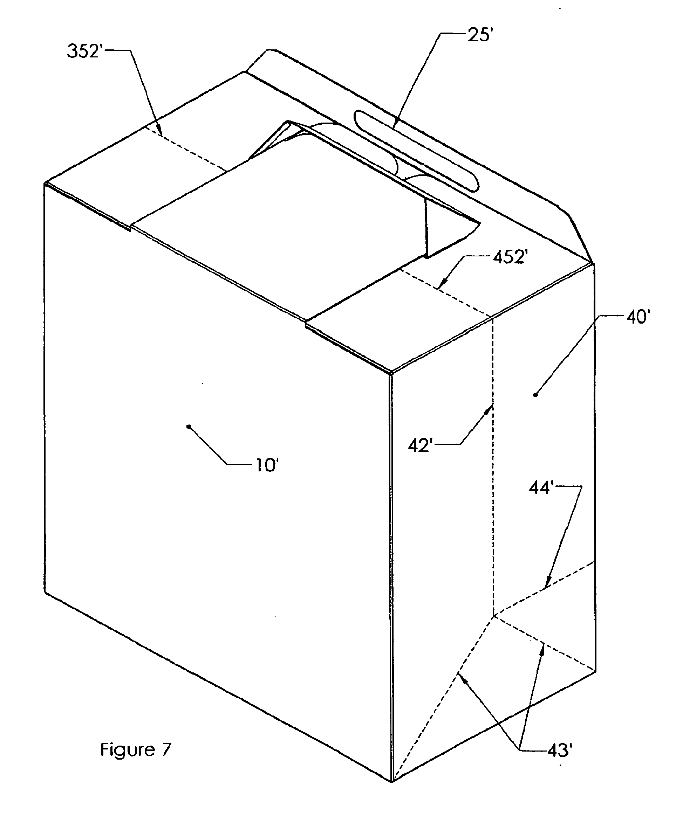

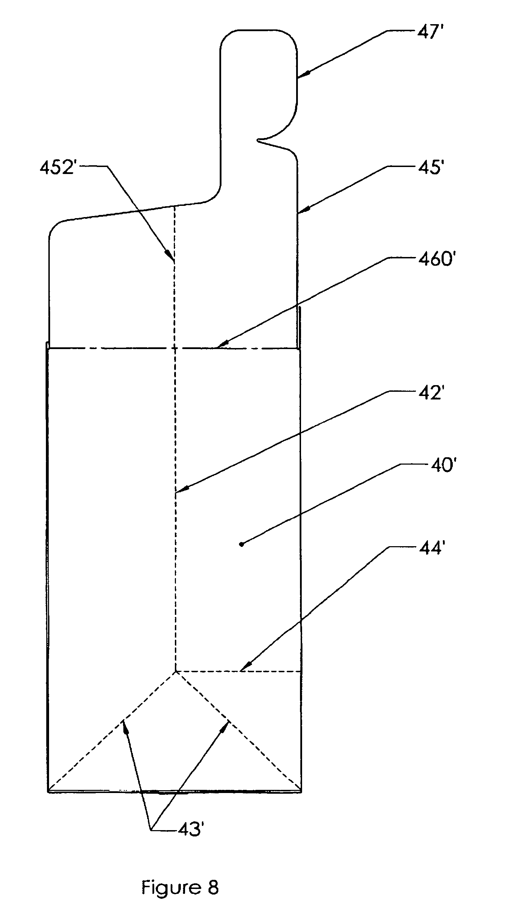

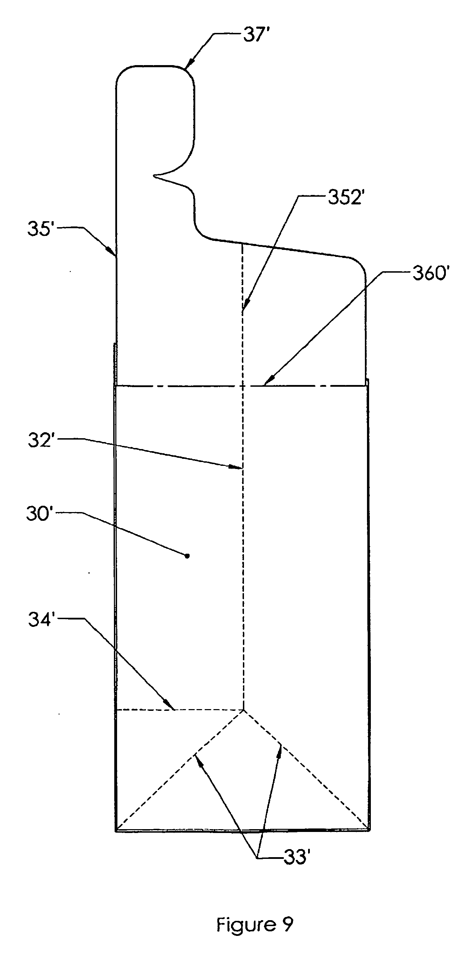

The second panel 20 is hingedly connected along one side to the third panel 30 and along the other side to a tab 20a. The blank is thus assembled into a box by connecting the tab 20a to the fourth panel 40 and folding in the sixth and eighth panels 52 and 54 of the third and fourth panels 30 and 40, followed by the fifth panel 51 connected to the second panel 20 and finally the seventh panel 53 connected to the first panel 10. The seventh panel 53 connected to the first panel 10 has a further fold line 57, which will overlap with the fifth panel 51 connected to the second panel 20. As shown in Figure 4, fifth panel 51 becomes a base panel 51', which connects with the further base panel 53' via an overlap caused by the folded line 57'. Figure 4a shows a detail of the overlap. Thus the fifth, sixth, seventh and eighth panels 51 to 54 will form the base panels 51' to 54' of the assembled box, while the third and fourth panels 30 and 40 will form the side panels 30' and 40' (Figures 8 & 9) of the assembled box. The second panel 20 forms the rear panel 20' (Figure 2) and the first panel 10 forms the front panel 10' (Figure 7).

It will be obvious to an addressee that the above configuration may vary: the second panel 20 may be connected to the fourth panel 40; additionally or alternatively, the fold line 57 may be on the fifth panel 51 connected to the second panel 20.

Again referring to Figure 1, the ninth panel 60 may be folded around the second panel 20 and connected with the first panel 10 via a tab 67 to form a top panel of the assembled box.

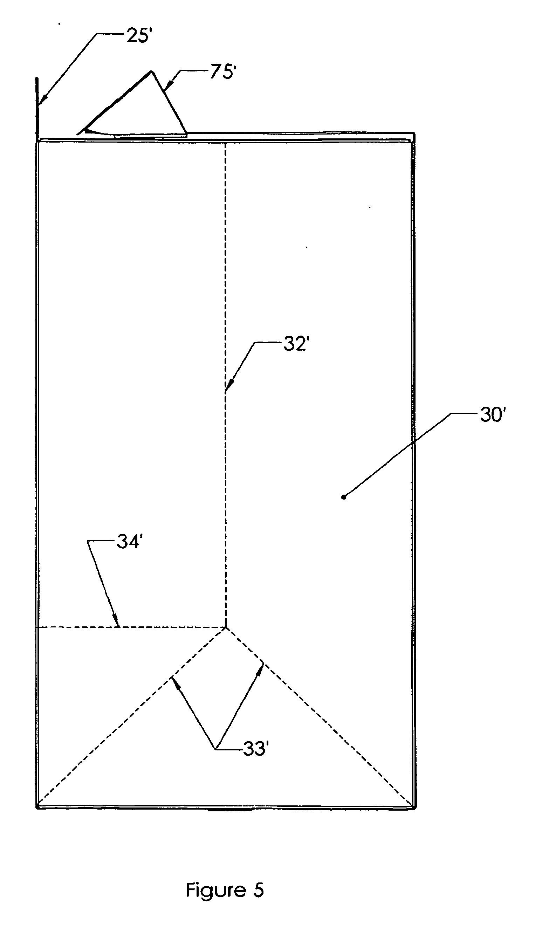

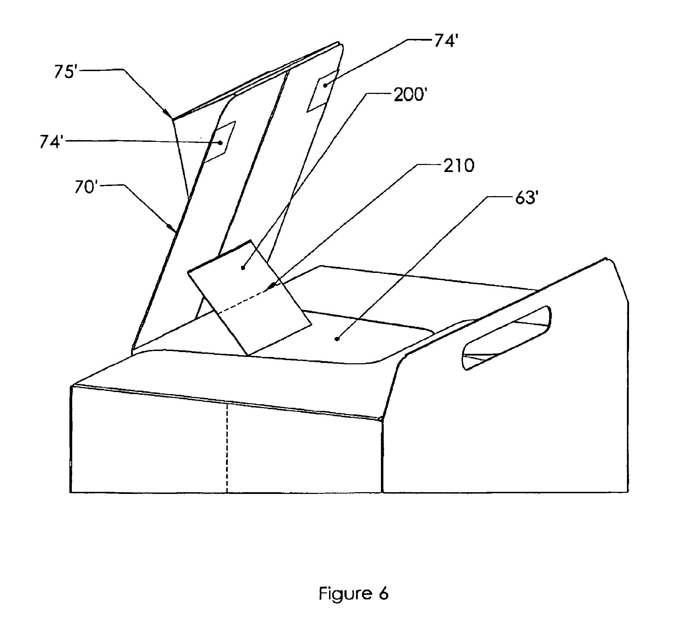

Referring now to Figures 1 and 6, where the ninth panel 60 is provided with a flap 63, the blank 100 may comprise a further tenth panel 70 hingedly connected with the first panel 10. The tenth panel 70 comprises portions 71 and 72, foldable around the tenth panel 70 thereby to strengthen it, together with a further portion 73 likewise foldable around the tenth panel 70, and another portion 75 foldable against the portion 73 and fixable thereto by adhesive. The adhesive may be part of the panel and covered by a strip (not shown) until the box is assembled. Two tabs 74 may then be folded around the portions 73, 75 and affixed to the tenth panel 70, thus forming a handle portion 75' of the assembled box, with the tenth panel 70 forming a lid 70' for the assembled box. Figure 5 shows the profile of the raised handle 75'.

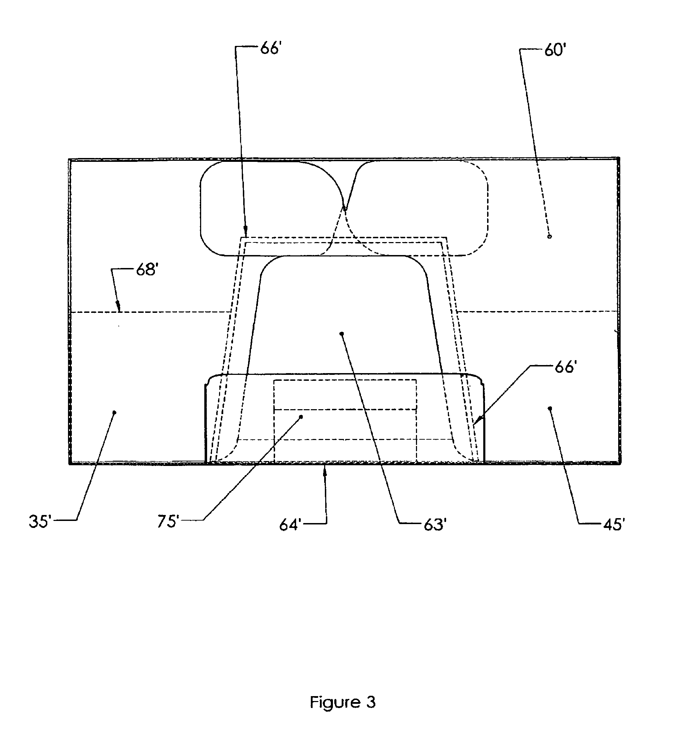

Referring now to Figure 6 in conjunction with Figure 3, the lid 70' of the assembled box is connectable with the flap 63' of the top panel via an interconnecting flange 200', which may have a fold line 210 across its width. Hence the lid 70' will cover the aperture 66' (shown in Figure 3) when the handle 75' is lowered, and the flap 63' will at least partially occlude the aperture 66' when the handle 75' is raised. In addition, the flange 200' will mean that the lid 70' can, in the closed position, act to limit occlusion of the aperture 66' by the flap 63'.

Such a box may therefore be used as a disposal box for e.g. sanitary waste without the contents being in view once disposed of. Conventional sanitary waste boxes are constructed of plastics materials and are not designed to be disposable; therefore, their use requires them to be collected or emptied on site. The present invention thus provides a disposable sanitary waste box, without the need for its being collected and/or emptied. Once the box of the present invention is filled, the lid panel 70' may be sealed down after use and the box may be disposed of. The box may be provided with a tape or other adhesive means for sealing the lid 70' to the box to prevent its opening.

Referring now to Figures 1, 5 and 7, the blank 100 is preferably made of card or other flexible resilient material, the blank 100 being scored or otherwise provided with further fold lines such that once assembled the box may be stored in a collapsed condition: the side panels 30' and 40' of the assembled box have fold lines 32', 42' along at least part of their length. The part 60 of the blank 100 which forms the top panel 60' has a fold line 68 across its width, while the part 20 of the blank 100 which forms the rear panel 20' has a fold line 22 across its width.

These fold lines mean that by folding the side panels 30' and 40' and the top panel, the front panel 10' and rear panel 20' may be brought into closer proximity, thus at least partially collapsing the assembled box.

Advantageously, and referring now to Figures 5 and 7, the side panels 30' and 40' are scored with respective fold lines 33'; 43' which extend divergently from respective fold lines 32'; 42' to approach the base of the box at an angle (best seen in Figure 5) so as to be coincident with the lowermost corners of each respective panel 30'; 40'. Each panel 30'; 40' also includes respective fold lines 34';44' which extend from the convergence of lines 33', 32'; 43', 42' horizontally across the respective side panel 30'; 40' to the fold line 22' across the width of the rear panel 20'. The respective fold lines 32', 33', 34'; 42', 43', 44' enable the base of the assembled box to be folded in the same plane as the front and rear panels 10' and 20', hence permitting further collapse of the box.

The card forming the blank may be printed or otherwise configured in a decorative pattern, or otherwise made to be aesthetically pleasing. The box may be treated with a fragrance and/or have anti-microbial properties, and/or be resistant to cleaning chemicals

Because card is resiliently deformable, once collapsed in this way the box may not easily revert to its original configuration, particularly after a long storage period whilst folded. Even when restored to its original configuration, the side panels 30' and 40' and rear panel 20' may in particular tend to collapse along their respective fold lines.

To overcome this and referring now to Figures 1, 8 and 9, the blank 100 is provided with eleventh and twelfth panels 35 and 45 which are foldable over the ninth panel 60 and which are hingedly connected to the third and fourth panels 30 and 40 of the blank 100.

The eleventh and twelfth panels 35 and 45 will form wing portions 35' and 45' of the assembled box, being foldable alongside the side panels 30' and 40' of the assembled box during storage.

As seen in Figure 3, the eleventh and twelfth panels 35' and 45' are shaped so that when overlying the ninth panel 60' (being the top panel 60' of the assembled box) they may partially occlude the aperture 66' to prevent the flap 63' from rising higher than the top panel 60' of the box. The eleventh and twelfth panels 35' and 45' may comprise complementarily-shaped interlockable portions 37' and 47' or be connectable by other means.

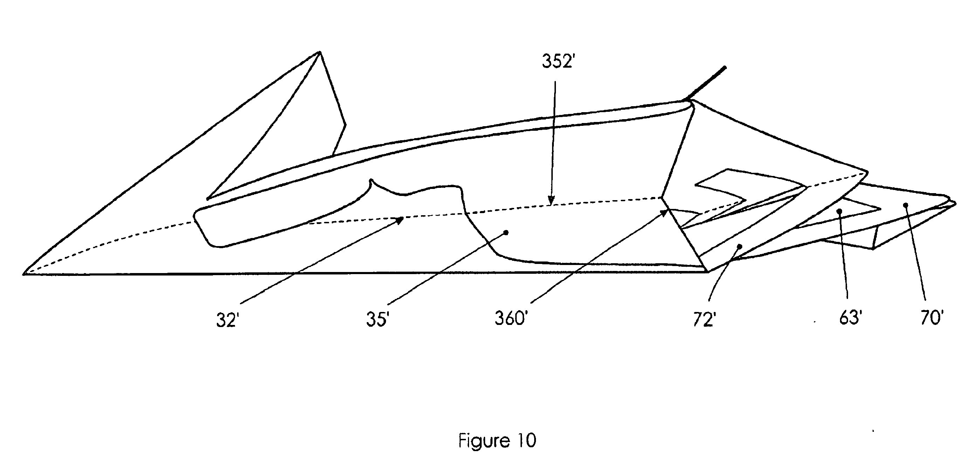

During storage of the assembled box, the wing portions 35' and 45' are folded back to lie alongside the side panels 30' and 40' (see Figure 10). Hence they have fold lines 352'; 452' along at least part of their length (shown in Figures 1, 8 and 9) which, in the folded condition (i.e. when wing portions 35'; 45' overlie respective panel 30'; 40'), overlie and can fold in the same direction as the fold lines 32'; 42' of the side panels 30' and 40' to enable the box to adopt a flat, folded configuration. In fact, because of the orientation and position of the fold lines 32', 352'; 42', 452', the wing portions 35'; 45' tend to snap into an overlying relation with the side panels 30', 40' when brought into proximity therewith (especially once the wing portions 35';45' and respective side panels 30'; 40' together describe an angle of less than 90°).

In constructing the box ready for use after storage, the wing portions 35' and 45' are folded away from the side panels 30' and 40', as seen in Figure 8 and Figure 9 against an urging force as described above. As the wing portions 35'; 45' are brought beyond the horizontal, the fold line 360'; 460' is brought into a straight condition. As the wing portion 35'; 45' is brought to the vertical (see Figures 8 and 9) the fold line 352'; 452' is directed in the opposite sense to the fold line 32'; 42' in the respective side panel 30'; 40' (i.e. the wing portion 35'; 45' will fold in to the plane of the paper as shown in Figures 8 and 9, whereas the panel 30'; 40' will fold out of the plane of the paper as shown in Figures 8 and 9). Because of the opposite direction of the fold lines 352', 32'; 452', 42' the fold line 360'; 460' is maintained in a straight or flat (as opposed to folded) configuration which helps to maintain the box in the erected state.

The wing portions 35' and 45' are interlocked by mutually engaging the cutouts therein. Once interlocked in that position, the interlock between the wing portions holds the wing portions 35' and 45' together, preventing them from returning to the folded state. Moreover, the wing portions 35' and 45' provide further residual stiffness to the assembled box. Of course the skilled person will recognise that the wing portions 35', 45' could be mutually engaged and secured using adhesive or other securing means, such as hook and eye fasteners. The adhesive may be covered by a release strip.

In unfolding up and around the top panel 60', the wing portions 35' and 45' will pass under the lid panel 70' but over the flap 63'. They must thus be shaped to permit access to the interior of the box via the aperture 66'. They may as previously discussed be shaped to further define edges of the aperture 66' and prevent the flap 63 from rising above the top panel of the assembled box.

Once assembled, the wing portions 35', 45' prevent the flap 63' from lifting up and out of overlying relations with the aperture 66'. However, the flap 63' may be pushed downwardly to allow deposit of articles within the box.

In certain embodiments, where the flap 63' is connected or secured to the lid 70', operation of the lid 70', e.g. from raised to lowered configuration, causes the flap 63' to move from an aperture occluding position to a non occluding position.
In use, the box will be assembled from a flat condition as indicated above. The lid 70' will naturally fall into the lowered configuration (see Figure 5). If a person wishes to deposit an article into the box they will raise the lid 70' using the handle 75' which will cause the flap 63' to be pivotally raised into an aperture occluding position (see Figure 6). An article (not shown) may then be deposited onto the flap 63' and the lid 70' lowered. As the lid 70' is lowered, preferably using the handle 75', the flap 63' is pivoted downwardly through the action of interconnecting flange 200' and/or the weight of the article and/or gravity to cause the article to be deposited within the box. The lid portion 70' will naturally adopt the lowered configuration thereby inhibiting access to, and sight of, the contents of the box. The use of the handle 75' and flap-operating lid 70' ensures that the user has to make minimal contact with the flap 63'.

In other embodiments the flap 63' may be urged towards the aperture occluding position by a further member, e.g. a member bearing against the underside of the flap connected to the inside of the box. In such a case, once a downward urging force is removed from the flap 63' it will naturally adopt its aperture 66' occluding position. In this way, the contents of the box are not viewable without applying a downward force to the flap 63'.

In which case the box will usually be provided with a lid portion so as to cover the flap 63' when not in use.

In such a manner the contents of the box are always isolated from the outside world and the user.

Once the box is full, or when a specified use period has expired the box is simply removed and a new box put in its place. The used box including the contents may be disposed of using best practice.

Clearly, such a disposable box has many benefits over conventional plastic bins, not least where the deposited items are soiled or include biological material, for example tampons and other female sanitary wear, nappies or diapers, incontinence devices, tissues, toilet paper, bandages, sutures, used dressings.

Various modifications of the box and blank may be considered without departing from the invention as defined in the claims.

## Claims

1. A collapsible box (100) for disposal of articles such as sanitary wear, the box comprising a base, at least three walls (10, 20, 30) upstanding therefrom and a top (60), the top having an aperture (66) through which items may be placed in the box and a movable flap (63) arranged to be able to at least partially occlude the aperture, a panel or wing portion being movable between a first condition for collapsing the box and a second condition at least partially overlying the top, **characterized in that** at least a part of the flap is engaged or engageable by the panel or wing portion (35) to inhibit upward motion thereof, and, in the second condition, the flap is at least partly overlying the aperture.

2. A collapsible box as claimed in Claim 1, further comprising a fourth wall (40) upstanding from the base.

3. A collapsible box according to claim 1 or 2, further comprising a second wing portion (45), interengageable with said first wing portion (35) in said second condition.

4. A collapsible box according to Claim 2 or 3, wherein the second wing portion is foldable from a first condition for collapsing the box and a second condition at least partially overlying the top.

5. A collapsible box according to any of Claims 1 - 4, further comprising a lid (70) arranged to be movable to cover the aperture.

6. A collapsible box according to Claim 4, wherein the lid is operable from a lowered configuration to a raised configuration and is operably connected to the flap such that when the lid is raised upwardly, said flap is urged upwardly.

7. A collapsible box as claimed in Claim 5 or 6, wherein the lid is secured to the flap.

8. A collapsible box as claimed in any one of Claims 1 to 7, wherein the lid comprises a handle (75).

9. A blank for forming a collapsible box according to claim 2, comprising:
a first panel (10) hingedly connected to a second panel (20) via a third panel (30);
a fourth panel (40) hingedly connected to the first panel or the second panel;
at least a fifth panel (51);
a tab (20a) permitting attachment of the fourth panel with the first or second panel to permit assembly of a box in which the first panel will form a front, the second a rear, the third and fourth the sides and the fifth panel the base of the assembled box;
the third and fourth panels being scored or otherwise foldable along at least a part of their length, wherein the blank further comprises a plurality of further panels (35, 45) hingedly connected to the third and fourth panels and moveable between a first position alongside the third and fourth panels and a second position foldable over the top of the assembled box, the further panels being shaped such that they can fixedly locate in the second position, **characterized in that** at least a part of the flap is engaged or engageable by the panel or wing portion (35) to inhibit upward motion thereof, and, in the second condition, the flap is at least partly overlying the aperture.

10. A blank as claimed in Claim 9, wherein the blank further comprises a further panel hingedly connected with the first or second panel and defining an aperture, this further panel being foldable around the first or second panel to form a top panel for the assembled box.

11. A blank as claimed in Claim 10, wherein the top-forming panel further comprises a flap at least partially occluding the aperture.

12. A blank as according to any one of Claims 9 - 11, wherein the further panel(s) is/are shaped such that in the second position they can partially define the aperture.

13. A blank as claimed in Claim 11 or Claim 12, wherein the blank further comprises a further panel hingedly connected with the second or first panel, this panel being foldable around the second or first panel to form a lid for the assembled box.

14. A box according to any one of claims 1 - 8 or a blank according to any one of Claims 9 to 13, wherein the material forming the blank is waterproof or otherwise treated to resist water and/or is treated to resist cleaning chemicals, and/or treated to have antimicrobial properties and/or treated with a fragrance material, and/or adapted to receive a fragrance emitter.

## Patentansprüche

1. Zusammenlegbare Schachtel (100) zur Entsorgung von Gegenständen wie Hygieneartikeln, wobei die Schachtel eine Basis, mindestens drei Wände (10, 20, 30), die davon hochstehen, und eine Oberseite (60) aufweist, wobei die Oberseite eine Öffnung (66), durch die hindurch Gegenstände in die Schachtel eingebracht werden können, und eine bewegliche Lasche (63) aufweist, die so angeordnet ist, dass sie die Öffnung zumindest teilweise verlegen kann, wobei ein Element oder ein Flügelabschnitt zwischen einem ersten Zustand, in dem die Schachtel zusammengelegt werden kann, und einem zweiten Zustand, wo es bzw. er zumindest zum Teil über der Oberseite liegt, bewegt werden kann, **dadurch gekennzeichnet, dass**
zumindest ein Teil der Lasche von dem Element oder dem Flügelabschnitt (35) gegriffen werden kann, um seine Aufwärtsbewegung zu hemmen, und die Lasche im zweiten Zustand zumindest zum Teil über der Öffnung liegt.

2. Zusammenlegbare Schachtel nach Anspruch 1, ferner eine vierte Wand (40) aufweisend, die von der Basis hochsteht.

3. Zusammenlegbare Schachtel nach Anspruch 1 oder 2, ferner einen zweiten Flügelabschnitt (45) aufweisend, der im zweiten Zustand mit dem ersten Flügelabschnitt (35) in gegenseitigen Eingriff gebracht werden kann.

4. Zusammenlegbare Schachtel nach Anspruch 2 oder 3, wobei der zweite Flügelabschnitt aus einem ersten Zustand, in dem die Schachtel zusammengelegt werden kann, und einem zweiten Zustand, in dem er zumindest teilweise über der Oberseite liegt, umgelegt werden kann.

5. Zusammenlegbare Schachtel nach irgendeinem der Ansprüche 1 - 4, ferner einen Deckel (70) aufweisend, der so angeordnet ist, dass er die Öffnung abdeckt.

6. Zusammenlegbare Schachtel nach Anspruch 4, wobei der Deckel so betätigt werden kann, dass er aus einer abgesenkten Konfiguration in eine angehobene Konfiguration bewegt wird, und wirkmäßig so mit der Lasche verbunden ist, dass die Lasche nach oben gedrängt wird, wenn der Deckel angehoben wird.

7. Zusammenlegbare Schachtel nach Anspruch 5 oder 6, wobei der Deckel an der Lasche festgelegt ist.

8. Zusammenlegbare Schachtel nach irgendeinem der Ansprüche 1 bis 7, wobei der Deckel einen Griff (75) aufweist.

9. Zuschnitt zum Ausbilden einer zusammenlegbaren Schachtel nach Anspruch 2, aufweisend:
ein erstes Element (10), das über ein drittes Element (30) gelenkig mit einem zweiten Element (20) verbunden ist;
ein viertes Element (40), das gelenkig mit dem ersten Element oder dem zweiten Element verbunden ist;
zumindest ein fünftes Element (51);
eine Lasche (20a), die eine Befestigung des vierten Elementes an dem ersten oder zweiten Element ermöglicht, um einen Zusammenbau der Schachtel zu ermöglichen, bei dem das erste Element eine Vorderseite, das zweite eine Rückseite bildet, das dritte und vierte die Seiten bilden und das fünfte Element die Basis der zusammengebauten Schachtel bildet;
wobei die dritten und vierten Elemente eingeritzt oder auf andere Weise entlang zumindest einem Teil ihrer Länge faltbar sind, wobei der Zuschnitt ferner eine Mehrzahl von weiteren Elementen (35, 45) aufweist, die gelenkig mit den dritten und vierten Elementen verbunden sind und zwischen einer ersten Position entlang der dritten und vierten Elemente und einer zweiten Position, in der sie über die Oberseite der zusammengebauten Schachtel gelegt werden können, bewegt werden können, wobei die weiteren Elemente so geformt sind, dass sie in der zweiten Position festgelegt werden können, **dadurch gekennzeichnet, dass**
zumindest ein Teil der Lasche von dem Element oder dem Flügelabschnitt (35) gegriffen werden kann, um seine Aufwärtsbewegung zu hemmen, und die Lasche im zweiten Zustand zumindest zum Teil über der Öffnung liegt.

10. Zuschnitt nach Anspruch 9, wobei der Zuschnitt ferner ein weiteres Element aufweist, das gelenkig mit dem ersten oder zweiten Element verbunden ist und eine Öffnung definiert, wobei dieses weitere Element um das erste oder zweite Element herum gelegt werden kann, um ein oberes Element für die zusammengebaute Schachtel zu bilden.

11. Zuschnitt nach Anspruch 10, wobei das die Oberseite bildende Element ferner eine Lasche aufweist, welche die Öffnung zumindest zum Teil verlegt.

12. Zuschnitt nach einem der Ansprüche 9 - 11, wobei das mindestens eine weitere Element so geformt ist, dass es in der zweiten Position die Öffnung zum Teil definieren können.

13. Zuschnitt nach Anspruch 11 oder Anspruch 12, wobei der Zuschnitt ferner ein weiteres Element aufweist, das gelenkig mit dem zweiten oder dem ersten Element verbunden ist, wobei dieses Element um das zweite oder das erste Element herum gelegt werden kann, um einen Deckel für die zusammengebaute Schachtel zu bilden.

14. Schachtel nach irgendeinem der Ansprüche 1 - 8 oder Zuschnitt nach irgendeinem der Ansprüche 9 bis 13, wobei das Material, aus dem der Zuschnitt besteht, wasserfest ist oder ansonsten so behandelt worden ist, dass es wasserbeständig ist, und/oder so behandelt worden ist, dass es gegenüber Reinigungschemikalien beständig ist, und/oder so behandelt worden ist, dass es antimikrobiotische Eigenschaften hat, und/oder mit einem Duftstoff behandelt worden ist, und/oder dafür ausgelegt ist, einen Duftspender aufzunehmen.

## Revendications

1. Boîte pliable (100) pour l'évacuation des articles, tels que des serviettes hygiéniques, la boîte comprenant une base, au moins trois parois (10, 20, 30) s'élevant verticalement depuis celle-ci et une partie supérieure (60), la partie supérieure ayant une ouverture (66) à travers laquelle les articles peuvent être placés dans la boîte et un clapet mobile (63) disposé de manière à au moins occlure partiellement l'ouverture, un panneau ou une partie d'ailette étant mobile entre un premier état de pliage de la boîte et un second état, au moins recouvrant partiellement la partie supérieure, **caractérisé en ce qu'**au moins une partie du clapet est enclenchée ou puisse être enclenchée par le panneau ou la partie d'ailette (35) pour bloquer le mouvement vers le haut de celui-ci, et, dans la second état, le clapet est superposé au moins partiellement sur l'ouverture.

2. Boîte pliable selon la revendication 1, comprenant en outre une quatrième paroi (40) s'élevant à partir de la base.

3. Boîte pliable, selon la revendication 1 ou 2, comprenant en outre une seconde partie d'ailette (45), interconnectable avec ladite première partie d'ailette (35) dans ledit second état.

4. Boîte pliable, selon la revendication 2 ou 3, dans lequel la seconde partie d'ailette est pliable à partir d'un premier état de pliage de la boîte et un second état au moins recouvrant partiellement la partie supérieure.

5. Boîte pliable selon l'une quelconque des revendications 1 à 4, comprenant en outre un couvercle (70) conçu pour être mobile pour couvrir l'ouverture.

6. Boîte pliable selon la revendication 4, dans lequel le couvercle peut être actionné à partir d'une configuration abaissée par rapport à une configuration soulevée étant raccordé de manière opérationnelle au clapet de telle sorte que lorsque le couvercle soit soulevé vers le haut, ledit clapet soit poussé vers le haut.

7. Boîte pliable selon la revendication 5 ou 6, dans lequel le couvercle est solidaire du clapet.

8. Boîte pliable selon l'une quelconque des revendications 1 à 7, dans lequel le couvercle comporte une poignée (75).

9. Flan pour former une boîte pliable selon la revendication 2, comprenant :
un premier panneau (10) relié de manière articulée à un second panneau (20) par l'intermédiaire d'un troisième panneau (30) ;
un quatrième panneau (40) relié de manière articulée au premier panneau ou au second panneau ; au moins un cinquième panneau (51) ;
une languette (20a) permettant la fixation du quatrième panneau avec le premier ou le second panneau pour permettre l'assemblage de la boîte dans laquelle le premier panneau formera un avant, le second un arrière, le troisième et le quatrième les côtés et le cinquième panneau formant la base de la boîte assemblée ;
les troisième et quatrième panneaux étant marqués ou autrement pliables le long d'au moins une partie de leur longueur, dans lequel le flan comprend en outre une pluralité d'autres panneaux (35, 45) reliés de façon articulée aux troisième et quatrième panneaux et mobiles entre une première position le long des troisième et quatrième panneaux et une seconde position pliable sur le dessus de la boîte assemblée, les autres panneaux ayant une forme leur permettant de se placer de manière fixe dans la deuxième position, **caractérisé en ce qu'**au moins une partie du clapet est enclenchée ou puisse être enclenchée par le panneau ou la partie d'ailette (35) pour bloquer le mouvement vers le haut de celui-ci, et, dans la second état, le clapet est superposé au moins partiellement sur l'ouverture.

10. Flan selon la revendication 9, dans lequel le flan comprend en outre un autre panneau relié de manière articulée avec le premier ou le second panneau et définissant une ouverture, cet autre panneau étant repliable le long du premier ou du second panneau pour former un panneau supérieur de la boîte assemblée.

11. Flan selon la revendication 10, dans lequel le panneau formant le dessus comprend en outre un clapet obturant au moins partiellement l'ouverture.

12. Flan selon l'une quelconque des revendications 9 à 11, dans lequel le panneau supplémentaire ou les panneaux supplémentaires ont une forme de telle sorte que dans la seconde position, ils peuvent définir partiellement l'ouverture.

13. Flan selon la revendication 11 ou la revendication 12, dans lequel le flan comprend en outre un autre panneau relié de manière articulée avec le deuxième ou le premier panneau, ce panneau u pouvant être plié le long du deuxième ou du premier panneau pour former un couvercle pour la boîte assemblée.

14. Boîte selon l'une quelconque des revendications 1 à 8 ou flan selon l'une quelconque des revendications 9 à 13, dans lequel la matière formant le flan est imperméable à l'eau ou autrement traitée pour résister à l'eau et/ou est traitée pour résister aux produits chimiques de nettoyage, et/ou traitée pour avoir des propriétés antimicrobiennes et/ou traitée avec une substance odorante, et/ou adaptée pour recevoir un émetteur de parfum.
